Europäisches Patentamt

European Patent Office

Office européen des brevets

(19)

(11) **EP 1 634 621 A1**

(12) **DEMANDE DE BREVET EUROPEEN**

(43) Date de publication:
**15.03.2006 Bulletin 2006/11**

(51) Int Cl.:
*A61Q 3/02* *(2006.01)*    *A61K 8/85* *(2006.01)*

(21) Numéro de dépôt: **05291352.2**

(22) Date de dépôt: **23.06.2005**

(84) Etats contractants désignés:
**AT BE BG CH CY CZ DE DK EE ES FI FR GB GR
HU IE IS IT LI LT LU MC NL PL PT RO SE SI SK TR**
Etats d'extension désignés:
**AL BA HR LV MK YU**

(30) Priorité: **20.08.2004  FR 0451879**

(71) Demandeur: **L'OREAL
75008 Paris (FR)**

(72) Inventeur: **Ilekti, Philippe
94700 Maison-Alfort (FR)**

(74) Mandataire: **Boulard, Denis
L'OREAL - D.I.P.I.
25-29 Quai Aulagnier
92600 Asnières (FR)**

(54) **Kit de maquillage ou de soin des ongles**

(57)    La présente invention a pour objet un kit de maquillage ou de soin des ongles comprenant, dans des conditionnements séparés :

i) une première composition liquide et
ii) un film polymérique souple, ledit film et ladite première composition liquide étant tels que lorsque le film est appliqué sur l'ongle préalablement revêtu de ladite première composition, le film adhère à l'ongle.

L'invention a également pour objet un procédé de maquillage ou de soin des ongles.

EP 1 634 621 A1

**Description**

La présente invention concerne un kit de maquillage et/ou de soin des ongles ou des faux ongles ainsi qu'un procédé de maquillage des ongles ou des faux ongles.

D'une manière générale, le maquillage des ongles ou des faux ongles est contraignant car il requiert un laps temps important. En effet, l'utilisatrice de vernis à ongles s'applique plusieurs couches de vernis qu'il faut laisser sécher. Au bout de 3 à 5 jours, le vernis s'écaille et la brillance diminue, l'utilisatrice de vernis à ongles doit se démaquiller et de se maquiller les ongles à nouveau.

En conséquence, dans un souci de proposer des compositions de maquillage des ongles, ou des faux ongles, dotées d'une meilleure tenue, des compositions de vernis à ongles sous forme de kit de deux compositions liquides de vernis à ongles ont été proposé. Cependant, la tenue reste moyenne et l'application de plusieurs couches reste encore plus contraignante.

De plus, lorsque le vernis à ongles est coloré, son application reste délicate et longue en raison du risque de débordement du vernis sur le contour de l'ongle.

La présente invention vise précisément à proposer un kit de maquillage et/ou de soin des ongles ou des faux ongles permettant de surmonter les inconvénients précités, c'est à dire présentant une tenue sur l'ongle dans le temps améliorée, qui soit facile et rapide à appliquer.

De façon plus précise, l'invention a pour objet un kit de maquillage ou de soin des ongles comprenant:

    i) une première composition liquide
    ii) un film polymérique souple, ledit film et ladite première composition liquide étant tels que lorsque le film est appliqué sur l'ongle préalablement revêtu de ladite première composition, le film adhère à l'ongle.

Selon un mode de réalisation, le kit de maquillage selon l'invention comprend le film polymérique et la première composition liquide dans des conditionnements séparés.

L'invention a encore pour objet l'utilisation d'un kit tel que décrit ci-dessus, pour obtenir un film déposé sur l'ongle, présentant des propriétés de tenue et/ou une résistance à l'usure améliorée.

Selon un autre aspect, la présente invention a également pour objet un procédé de maquillage des ongles caractérisé en ce qu'il consiste :

    a) à appliquer sur l'ongle au moins une couche d'une première composition liquide,
    b) puis à appliquer sur ladite couche un film polymérique souple apte à adhérer sur l'ongle via ladite première composition liquide.

Selon une variante, l'invention a encore pour objet un procédé de maquillage des ongles consistant à appliquer sur l'ongle un film polymérique souple dont la face destinée à être en contact avec l'ongle a été préalablement enduite d'au moins une couche d'une première composition liquide, ledit film et ladite première composition liquide étant tels que lorsque le film est appliqué sur l'ongle préalablement revêtu de ladite première composition, le film adhère à l'ongle.

Le film polymérique souple, et en particulier l'excès de film, peut être prédécoupé ou découpé, avant ou après son application, selon la taille et la forme désirée avec de petits ciseaux, un coupe ongles ou en grattant le film.

Selon un mode de réalisation, une couche supplémentaire d'une seconde composition liquide, tel qu'un vernis à ongles conventionnel, comprenant un polymère filmogène et un milieu solvant organique, ou « top coat » est appliquée sur ledit film polymérique afin d'améliorer la brillance de celui-ci .

Le film polymérique du kit conforme à l'invention est souple et présente une flexibilité suffisante pour se conformer au profil de l'ongle. Il se distingue en cela d'un faux ongle qui ne présente pas une telle flexibilité.

Au sens de la présente invention, le terme « souple » qualifie une flexibilité suffisante du film polymérique du kit selon l'invention. Plus précisément, ce film souple est apte à se prêter à des déformations mécaniques de type étirement pour s'ajuster à la surface d'un ongle. Cette déformabilité est notamment caractérisée par le paramètre de déformation à la rupture $\varepsilon_r$ discuté ci-après.

Le film polymérique souple selon l'invention se différencie d'un article de type faux ongle qui se caractérise par une rigidité incompatible avec une telle déformation mécanique.

Une autre différence entre le film polymérique souple conforme à l'invention et un faux ongle, réside dans la sensibilité de ce film vis-à-vis des solvants organiques polaires du type acétone, ester et/ou alcool court. En effet, le film polymérique possède une aptitude à gonfler, et qui notamment peut se traduire par une augmentation de son poids lorsqu'il est mis en contact avec l'un de ces solvants. Un faux ongle est totalement dénué d'une telle sensibilité. Cette aptitude à gonfler est précisément avantageuse pour son élimination lorsque celui-ci est appliqué en surface d'un ongle ou d'un faux ongle. En effet, le film polymérique peut être aisément éliminé par simple démaquillage à l'aide d'un dissolvant classique, par opposition à un faux ongle qui doit être retiré. Plus précisément, il peut être démaquillé par des solvants organiques et notamment par les acétates d'alkyle et leurs mélanges.

Il présente également une tenue dans le temps significative et notamment à l'échelle d'au moins 5 jours, de préférence une semaine. Il s'avère ainsi résistant à l'eau, aux frottements et aux chocs, et ne présente pas d'usure ni d'écaillage significatifs dans ce délai.

Selon un mode de réalisation, le film polymérique souple conforme à la présente invention peut se présenter sous diverses formes telles qu'une étoile, un carré, un rond, etc....

## I/ Première composition liquide

Selon un premier mode de réalisation, la première composition comprend un matériau adhésif et une phase solvant, comprenant de préférence au moins un solvant organique.

### a) Matériau adhésif

Par « matériau », on entend au sens de la présente invention un polymère ou un système polymérique pouvant comprendre un ou plusieurs polymères de natures différentes. Ce matériau adhésif peut se présenter sous forme d'une solution de polymère ou d'une dispersion de particules de polymères dans un solvant. Ce matériau adhésif peut en outre contenir un plastifiant comme défini ci-dessous. Ce matériau adhésif doit présenter un certain pouvoir collant défini par ses propriétés viscoélastiques.

Les propriétés viscoélastiques d'un matériau sont classiquement définies par deux valeurs caractéristiques qui sont les suivantes :

le module élastique qui représente le comportement élastique du matériau pour une fréquence donnée et qui est classiquement noté G',

le module visqueux qui représente le comportement visqueux du matériau pour une fréquence donnée et qui est classiquement noté G".

Ces grandeurs sont notamment définies dans le "Handbook of Pressure Sensitive Adhesive Technology" 3rd edition, D. Satas, chap. 9, p. 155 à 157.

Les matériaux adhésifs utilisables selon la présente invention présentent des propriétés viscoélastiques qui sont mesurées à une température de référence de 35 °C et dans un certain intervalle de fréquences.

Dans le cas de matériaux adhésifs sous forme de solution ou de dispersion de polymère dans un solvant volatil (tel que l'eau, un ester court, un alcool court, de l'acétone ... ), on mesure les propriétés viscoélastiques de ce matériau dans des conditions dans lesquelles il présente une teneur en solvant volatil inférieure à 30 %, et en particulier une teneur en solvant volatil inférieure à 20 %.

On mesure en particulier le module élastique du matériau à trois fréquences différentes :

à faible fréquence, soit à $2.10^{-2}$ Hz,
à une fréquence intermédiaire, soit à 0,2 Hz,
à haute fréquence, soit à 2 Hz,
et le module visqueux à la fréquence de 0,2 Hz.

Ces mesures permettent d'évaluer l'évolution du pouvoir collant du matériau adhésif au cours du temps.

Ces propriétés viscoélastiques sont mesurées lors d'essais dynamiques sous sollicitations sinusoïdales de faible amplitude (petites déformations) réalisés à 35 °C sur une plage de fréquence allant de $2.10^{-2}$ à 20 Hz sur un rhéomètre de type "Haake RS50®" sous une sollicitation de torsion/cisaillement, par exemple en géométrie cône-plan (par exemple avec un angle du cône de 1°).

Avantageusement, ledit matériau adhésif répond aux conditions suivantes :

$G'$(2 Hz, 35 °C) $\geq 10^3$ Pa, et
$G'$(35 °C) $\leq 10^8$ Pa, en particulier $G'$(35 °C) $\leq 10^7$ Pa,
$G'$($2.10^{-2}$ Hz, 35 °C) $\leq 3.10^5$ Pa,
dans lesquelles :

$G'$(2 Hz, 35 °C) est le module élastique de cisaillement dudit matériau adhésif, mesuré à la fréquence de 2 Hz et à la température de 35 °C,
$G'$(35 °C) est le module élastique de cisaillement dudit matériau adhésif, mesuré à la température de 35 °C, pour toute fréquence comprise entre $2.10^{-2}$ et 2 Hz,

G'(2.10$^{-2}$ Hz, 35 °C) est le module élastique de cisaillement dudit matériau adhésif, mesuré à la fréquence de 2.10$^{-2}$ Hz et à la température de 35 °C.

Dans une forme particulière de l'invention, le matériau adhésif répond également à la condition suivante :

- G"/G' (0,2 Hz, 35 °C) ≥ 0,35.

dans laquelle :

G"(0,2 Hz, 35 °C) est le module visqueux de cisaillement dudit matériau adhésif, mesuré à la fréquence de 0,2 Hz et à la température de 35 °C,
G'(0,2 Hz, 35 °C) est le module élastique de cisaillement dudit matériau adhésif, mesuré à la fréquence de 0,2 Hz et à la température de 35 °C.

Dans une forme particulière de l'invention, on a :

- G'(2 Hz, 35 °C) ≥ 5.10$^3$ Pa, et en particulier, G'(2 Hz, 35 °C) ≥ 10$^4$ Pa.

Dans une autre forme particulière de l'invention, on a :

- G'(2.10$^{-2}$ Hz, 35°C) ≤ 5.10$^4$ Pa.

En particulier, les matériaux adhésifs selon l'invention répondent aux quatre conditions suivantes :

G'(2 Hz, 35 °C) ≥ 10$^4$ Pa, et
G'(35 °C) ≤ 10$^8$ Pa, en particulier G'(35 °C) ≤ 10$^7$ Pa,
G'(2.10$^{-2}$ Hz, 35 °C) ≤ 5.10$^4$ Pa, et
G"/G'(0,2 Hz, 35 °C) ≥ 0,35.

Plus particulièrement, les matériaux adhésifs selon l'invention peuvent être choisis parmi les adhésifs de type "Pressure Sensitive Adhesives" (adhésifs sensibles à la pression) par exemple, comme ceux cités dans le "Handbook of Pressure Sensitive Adhesive Technology" 3$^{rd}$ édition, D. Satas.
Les matériaux adhésifs selon l'invention sont notamment des polymères choisis parmi les copolymères blocs ou statistiques comprenant au moins un monomère ou une association de monomères dont le polymère résultant à une température de transition vitreuse inférieure à la température ambiante (25 °C), ces monomères ou associations de monomères pouvant être choisis parmi le butadiène, l'éthylène, le propylène, l'isoprène, l'isobutylène, une silicone, et leurs mélanges. Des exemples de tels matériaux sont les polymères blocs de type styrène-butadiène-styrène, styrène-(éthylène-butylène)-styrène, styrène-isoprène-styrène comme ceux vendus sous les dénominations commerciales "Kraton®" de SHELL CHEMICAL Co. ou de "Vector®" d'EXXON.
Les matériaux adhésifs selon l'invention sont en particulier des polymères adhésifs choisis parmi :

les polyuréthannes,
les polymères acryliques,
les silicones,
les gommes butyliques, notamment parmi les polyisobutylènes,
les polymères éthylène-acétate de vinyle,
les polyamides éventuellement modifiés par des chaînes grasses,
les gommes naturelles,
et leur mélanges.

Il peut en particulier s'agir de copolymères adhésifs dérivant de la copolymérisation de monomères vinyliques avec des entités polymériques comme par exemple ceux décrits dans le brevet US 6 136 296. Sont également susceptibles de convenir à l'invention, les copolymères adhésifs décrits dans le brevet US 5 929 173 possédant un squelette polymérique, de Tg variant de 0°C à 45°C, greffés par des chaînes dérivant de monomères acryliques et/ou méthacryliques et possédant en revanche une Tg variant de 50 °C à 200 °C. Les matériaux adhésifs sont par exemple choisis parmi les polyisobutylènes présentant une masse molaire relative Mv supérieure ou égale à 10 000 et inférieure ou égale à 150 000. En particulier, cette masse molaire relative est supérieure ou égale à 18 000 et inférieure ou égale à 150 000. Comme produits commerciaux convenant particulièrement bien à la présente invention, on peut citer les polyisobutylènes de masses molaires relatives Mv respectives 40 000, 55 000 et 85 000 vendus sous les dénominations commerciales respectives "Oppanol B 10®", "Oppanol B 12®" et "Oppanol B 15®" par la société BASF, et leurs mélanges.

Selon un second mode de réalisation, la première composition est une composition de vernis à ongles liquide comprenant une phase solvant organique et/ou aqueuse et au moins un polymère filmogène.

### b) Polymère filmogène

Par polymère filmogène, on entend un polymère apte à former à lui seul ou en présence d'un agent auxiliaire de filmification, un film macroscopiquement continu sur un support, par exemple les matières kératiniques.

Le polymère filmogène peut être choisi parmi les polymères filmogène cités plus loin dans la description du film polymérique.

De préférence, le polymère filmogène est choisi parmi les celluloses et dérivés cellulosiques comme les nitrocelluloses et/ou les esters de cellulose tels que les acétates de cellulose, les propionates de cellulose, les butyrates de cellulose, les acétopropionates de cellulose et les acétobutyrates de cellulose.

Le polymère filmogène peut représenter de 1% à 70% en poids en matières sèches de polymère, par rapport au poids total de la première composition, mieux de 5% à 50%, et mieux de 10 à 45% en poids.

La première composition peut également comprendre un auxiliaire de filmification tel que décrit plus loin.

La première composition comprend avantageusement une phase solvant organique liquide comprenant au moins un solvant organique. A titre de solvant organique utilisable dans la composition selon l'invention on peut citer :

- les esters à chaîne courte (ayant de 3 à 8 atomes de carbone au total), tels que l'acétate d'éthyle, l'acétate de méthyle, l'acétate de propyle, l'acétate de n-butyle, l'acétate d'isopentyle ;
- les cétones liquides à température ambiante, telles que la méthyléthylcétone, la méthylisobutylcétone, la diisobutylcétone, l'isophorone, la cyclohexanone, l'acétone ;
- les alcools liquides à température ambiante, tels que l'éthanol, l'isopropanol, le diacétone alcool, le 2-butoxyéthanol, le cyclohexanol ;
- les glycols liquides à température ambiante, tels que l'éthylène glycol, le propylène glycol, le pentylène glycol, le glycérol ;
- les éthers de propylène glycol liquides à température ambiante, tels que le monométhyléther de propylène glycol, l'acétate de monométhyl éther de propylène glycol, le mono n-butyl éther de dipropylène glycol ;
- les aldéhydes liquides à température ambiante, tels que le benzaldéhyde, l'acétaldéhyde ;
- les carbonates tel que le carbonate de propylène, le carbonate de diméthyle ;
- les acétals tels que le méthylal ; et
- leurs mélanges.

De préférence, le solvant est un solvant volatil choisi parmi les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle, et leurs mélanges. Généralement, la phase solvant organique (solvant organique ou mélange de solvants organiques) liquide représente de 5 à 95% du poids total de la composition, de préférence de 10% à 85% en poids.

La première composition selon l'invention peut en outre comprendre une phase aqueuse constituée d'eau et éventuellement de solvants hydrosolubles.

La phase aqueuse peut dans ce cas représenter de 5 à 95% en poids par rapport au poids total de la composition, de préférence de 10% à 85% en poids.

Selon un mode de réalisation de l'invention, la première composition est transparente

Selon une variante, la première composition est exempte de matière colorante et de matériau à effet optique et/ou relief tels que décrit plus loin.

Avantageusement, la première composition liquide et le film polymérique souple présentent une compatibilité grâce à leur nature chimique et leur composition. En effet, dans un mode de réalisation particulier, le solvant majoritaire organique de la première composition est susceptible de conduire à une augmentation de masse du film polymérique souple mis à son contact, notamment d'au moins 10%, de préférence 20%, après immersion pendant 60 minutes dudit film dans ledit solvant à température ambiante (25°C). En d'autres termes, cette augmentation se traduit par une reprise en poids du film.

La mesure s'effectue selon le protocole suivant :

Des morceaux d'environ 1 cm$^2$ découpés dans le film polymérique souple sont pesés (mesure de la masse M1) puis immergés dans le solvant organique pendant 60 minutes ; après immersion, le morceau de film est essuyé pour éliminer l'excédent de solvant en surface puis pesé (mesure de la masse M2). La différence M2 — M1 correspond à la quantité de solvant absorbée par le film.

De préférence le film solide est soluble dans ledit solvant organique.

D'une manière générale, la première composition et le film polymérique souple sont tels que ledit film polymérique préalablement revêtu de la première composition puis appliqué à la surface d'un ongle synthétique ou naturel, ou appliqué sur un ongle synthétique ou naturel revêtu de la première composition, ne peut être ôté par pelage après au moins 24 heures de pose.

## II/ Film polymérique

Avantageusement, le film polymérique souple dérive de la réticulation d'une composition réticulable et/ou de l'évaporation de la phase solvant, organique ou aqueuse, d'une solution ou dispersion d'au moins un polymère filmogène.

### Extrait sec

Le film polymérique souple du kit selon l'invention est un film non liquide qui peut se caractériser par un extrait sec élevé. En effet il peut posséder une quantité de matière sèche supérieure à 80%, et en particulier supérieure à 85 % et en particulier supérieure à 90 % en poids par rapport à son poids total. Autrement dit la quantité de solvant volatil est inférieure à 20 %, en particulier inférieure à 15 % et plus particulièrement inférieure à 10 % en poids par rapport au poids total du film polymérique.

De préférence, la quantité de matière sèche, communément appelée « extrait sec » des films selon l'invention, est mesurée par échauffement de l'échantillon par des rayons infrarouges de 2 $\mu$m à 3,5 $\mu$m de longueur d'onde. Les substances contenues dans lesdits films qui possèdent une pression de vapeur élevée, s'évaporent sous l'effet de ce rayonnement. La mesure de la perte de poids de l'échantillon permet de déterminer « l'extrait sec » du film. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant.

On dépose environ 10 g de l'échantillon sur une coupelle métallique. Celle-ci, après introduction dans le dessiccateur, est soumise à une consigne de température de 120° C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

$$\text{Extrait Sec} = 100 \times (\text{masse sèche} / \text{masse humide}).$$

Selon une variante, le film polymérique n'est pas totalement sec, il est dit partiellement sec.

Au sens de la présente invention, le terme partiellement sec entend qualifier le fait que le film polymérique obtenu après évaporation de la phase solvant, organique ou aqueuse, d'une solution ou dispersion d'au moins un polymère filmogène, ou par réticulation d'une composition réticulable, n'est pas totalement exempt du solvant résiduel. En particulier, il possède une teneur en matière sèche inférieure à 80 %, en particulier inférieure à 75 % et plus particulièrement inférieure à 70 % en poids par rapport à son poids total.

Selon un mode de réalisation particulier, le film polymérique du kit selon l'invention se présente dans un réservoir, comme par exemple une poche, souple ou non, apte à contenir de manière étanche un produit. Il est en particulier imperméable à l'air et/ou aux solvants. Ce conditionnement permet de préserver ledit film d'un séchage total et prématuré avant son utilisation.

### Reprise à l'eau

Le film polymérique souple du kit selon l'invention peut se caractériser à l'état sec par une reprise à l'eau portée à 25 °C inférieure ou égale à 20 %, notamment inférieure ou égale à 16 %, et en particulier inférieure à 10 %.

Selon la présente demande, on entend par "reprise à l'eau du film", le pourcentage d'eau absorbé par le film polymérique après 60 minutes d'immersion dans l'eau, à 25 °C (température ambiante). La reprise à l'eau est mesurée pour des morceaux d'environ 1 cm$^2$ découpés dans le film polymérique sec qui sont pesés (mesure de la masse M1) puis immergés dans l'eau pendant 60 minutes ; après immersion, le morceau de film est essuyé pour éliminer l'excédent d'eau en surface puis pesé (mesure de la masse M2). La différence M2 — M1 correspond à la quantité d'eau absorbée par le film. La reprise à l'eau est égale à [(M2 — M1) / M1] x 100 et est exprimée en pourcentage de poids par rapport au poids du film.

Module de conservation E'

Par ailleurs, le film polymérique du kit selon l'invention présente avantageusement un module de conservation E' supérieur ou égal à 1 MPa, notamment allant de 1 MPa à 5000 MPa, en particulier supérieur ou égal à 5 MPa, notamment allant de 5 à 1000 MPa, et plus particulièrement supérieur ou égal à 10 MPa par exemple allant de 10 à 500 MPa à une température de 30 °C et une fréquence de 0,1 Hz.

La mesure du module de conservation est effectuée par DMTA (Dynamical and Mechanical Temperature Analysis ou Analyse dynamique et mécanique en température).

On effectue des essais de viscoélasticimétrie avec un appareil DMTA de Polymer TA Instruments (modèle DMA2980), sur un échantillon de film polymérique. On découpe (par exemple à l'emporte-pièce) les éprouvettes. Celles-ci ont typiquement une épaisseur d'environ 150 $\mu$m, une largeur de 5 à 10 mm et une longueur utile d'environ 10 à 15 mm. Les mesures sont effectuées à une température constante de 30°C.

L'échantillon est sollicité en traction et en petites déformations (on lui impose par exemple un déplacement sinusoïdal de $\pm$ 8 $\mu$m) lors d'un balayage en fréquence, la fréquence allant de 0,1 à 20 Hz. On travaille ainsi dans le domaine linéaire, sous de faibles niveaux de déformation.

Ces mesures permettent de déterminer le module complexe $E^* = E' + iE''$ du film de composition testé, E' étant le module de conservation et E'' le module dit de perte.

Déformation et/ou Energie à la rupture

Avantageusement, le film polymérique souple selon l'invention, possède à l'état sec une déformation à la rupture $\varepsilon_r$ supérieure ou égale à 5 %, notamment allant de 5 à 500 %, de préférence supérieure ou égale à 15 %, notamment allant de 15 à 400 % et/ou une énergie à rupture par unité de volume $W_r$ supérieure ou égale à 0,2 J/cm$^3$, notamment allant de 0,2 à 100 J/cm$^3$, de préférence supérieure à 1 J/cm$^3$, notamment allant de 1 à 50 J/cm$^3$.

La déformation à la rupture et l'énergie à rupture par unité de volume sont déterminées par des essais de traction effectués sur un film polymérique souple d'environ 200 $\mu$m d'épaisseur.

Pour effectuer ces essais, le film est découpé en éprouvettes haltères de longueur utile 33 $\pm$ 1 mm et de largeur utile 6 mm. La section (S) de l'éprouvette est alors définie comme : S = largeur x épaisseur (cm$^2$) ; cette section sera utilisée pour le calcul de la contrainte.

Les essais sont réalisés, par exemple, sur un appareil de traction commercialisé sous l'appellation Lloyd® LR5K. Les mesures sont réalisées à température ambiante (20 °C).

Les éprouvettes sont étirées à une vitesse de déplacement de 33 mm/min, correspondant à une vitesse de 100 % d'allongement par minute.

On impose donc une vitesse de déplacement et on mesure simultanément l'allongement $\Delta L$ de l'éprouvette et la force F nécessaire pour imposer cet allongement. C'est à partir de ces données $\Delta L$ et F que l'on détermine les paramètres contraintes $\sigma$ et déformation $\varepsilon$.

Il est ainsi obtenu une courbe contrainte $\sigma$ = (F/S) en fonction de la déformation $\varepsilon = (\Delta L/L_o) \times 100$, l'essai étant conduit jusqu'à rupture de l'éprouvette, $L_o$ étant la longueur initiale de l'éprouvette.

La déformation à la rupture $\varepsilon_r$ est la déformation maximale de l'échantillon avant le point de rupture (en %).

L'énergie à rupture par unité de volume $W_r$ en J/cm$^3$ est définie comme la surface sous cette courbe contrainte/déformation telle que :

$$W_r = \int_0^{\varepsilon_r} \sigma.\varepsilon.d\varepsilon$$

Le film polymérique du kit de maquillage selon l'invention peut être obtenu par réticulation d'une composition réticulable et/ou par évaporation de la phase solvant organique ou aqueuse d'une solution ou dispersion d'au moins un polymère filmogène.

**a) Film réticulé**

Au sens de la présente invention, un film qualifié de réticulé peut être totalement ou partiellement réticulé. Dans le cas d'une réticulation partielle, celle-ci est bien entendu suffisante pour former le film attendu.

Bien entendu, les composés mis en présence sont choisis, notamment selon la nature des fonctions qu'ils possèdent respectivement, pour être capable d'interagir dans les conditions de la réaction de réticulation considérée.

Cette réticulation peut ainsi être réalisée par voie thermique, photochimique et/ou chimique, en présence ou non d'un catalyseur. La réalisation de cette réticulation relève des compétences de l'homme de l'art.

- Selon une première variante, la réaction de réticulation s'apparente à une réaction de polyaddition ou polycondensation réalisée en présence ou en absence de catalyseur.

Selon cette première variante, le film organique peut notamment dériver de la réticulation d'un système réactif formé par :

au moins un premier composé (A) comprenant au moins deux fonctions (X), et
au moins un deuxième composé (B) comprenant au moins deux fonctions (Y), réactives vis-à-vis des fonctions X.

Avantageusement, le système réactif possède une fonctionnalité moyenne (nombre total des fonctions X et Y/nombre total des molécules de composés (A) et (B)) supérieure à 2 de manière à procurer un réseau tridimensionnel.

Plus particulièrement, pour l'obtention d'un effet de réticulation satisfaisant, la fonctionnalité moyenne du système réactif peut être au moins égale à 2,2 et plus particulièrement varier de 2,5 à 100.

Les composés (A) et (B) peuvent être d'origine organique et notamment de type monomère, oligomère, polymère et/ou copolymère ou de nature inorganique à l'image par exemple d'une particule minérale, auquel cas ils possèdent en surface les deux fonctions (X) ou (Y) requises.

Les fonctions X et Y réactives les unes vis-à-vis des autres sont choisies parmi des fonctions dites réactives et des fonctions comportant au moins un hydrogène labile.

Plus précisément, les fonctions réactives sont choisies parmi les fonctions isocyanates, époxydes et les doubles liaisons éthyléniques et les fonctions à hydrogène(s) labile(s) sont du type carboxylique, alcool notamment phénolique, amine primaire ou secondaire, amide, aminoalcool et/ou thiol.

Selon cette variante, les composés (A) et (B) mis en présence, possèdent respectivement au moins deux fonctions dites réactives de type époxyde et/ ou isocyanate et au moins deux fonctions à hydrogène(s) labile(s) notamment de type amine ou aminoalcool et peuvent notamment être choisis parmi les composés cités précédemment.

Par exemple, X peut être une fonction époxyde et/ou isocyanate et Y peut être choisi parmi une fonction acide carboxylique et/ou une fonction anhydride et/ou une fonction amine et/ou une fonction thiol et/ou une fonction hydroxyle, en particulier phénolique.

Dans cette variante de l'invention, la réticulation peut être réalisée en mettant en présence des composés (A) et/ou (B) possédant des fonctions (X) et/ou (Y) sous une forme bloquée et susceptible d'être débloquée préalablement ou dans les conditions réactionnelles retenues pour la réticulation. Cette alternative est bien connue de l'homme de l'art et ne sera pas décrite en détail.

Les composés à fonctions isocyanates :

Les composés comportant au moins deux fonctions isocyanate libres sont connus dans la technique. Il peut s'agir de polyisocyanates, y compris des diisocyanates ou triisocyanates, pouvant avoir une masse moléculaire inférieure à 500 000, voire inférieure à 10 000. Ces polyisocyanates sont généralement obtenus par polyaddition, polycondensation et/ou greffage, portant au moins deux fonctions isocyanates, soit aux extrémités de chaîne soit sur des groupes latéraux.

Les polyisocyanates peuvent être linéaires, ramifiés, aliphatiques, cycloaliphatiques ou aromatiques.

Comme polyisocyanate, on peut en particulier utiliser le DESMODUR® N de la société BAYER, le TOLONATE® HDB-LV de la société RHODIA.

Les composés à fonctions époxydes :

Les composés comportant au moins deux fonctions époxydes sont également connus de l'état de la technique. Ils peuvent être de toute nature chimique. Il peut s'agir de diépoxydes ou de polyépoxydes de faibles masses (inférieures ou égales à 5000), ou bien d'oligomères ou de polymères de toute nature chimique, obtenus par polyaddition, polycondensation et/ou greffage, portant au moins deux fonctions époxydes libres, soit aux extrémités de chaînes, soit en groupes latéraux.

Des polymères à fonctions époxy sont commercialisés sous les dénominations CYRACURE® UVR-6110, CYRACURE® UVR-6105 , CYRACURE® ERL-4221E, CYRACURE® ERL-4206 , CYRACURE® UVR 6128 , CYRACURE® UVR 6216 par la société UNION CARBIDE, DER® 439 par la société DOW CHEMICAL, les EPIKATES® 828,1001,1004,1007 de la société SHELL, l'ARALDITE® ECN1299 de la société CIBA-GEIGY, les EPOXYNOVOLACS® de la société DOW CHEMICAL.

Les composés à doubles liaisons éthyléniques :

Les composés portant des doubles liaisons éthyléniques peuvent être de toute nature chimique. Ils peuvent notamment être choisis parmi :

les polyesters à groupes (méth)acrylate latéraux et/ou terminaux :

De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule).

les polyuréthannes et/ou polyurées à groupes (méth)acrylate notamment obtenus par polycondensation :

De tels polyuréthannes/polyurées à groupes acrylates sont commercialisés par exemple sous la dénomination SR 368 (tris(2-hydroxyéthyl)isocyanurate-triacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse molaire 800, 6 fonctions acrylate par molécule).
On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422 IRR® 424 par la société UCB.

les polyéthers à groupes (méth)acrylate obtenus par estérification, par l'acide (méth)acrylique, des groupes hydroxyle terminaux d'homopolymères ou de copolymères d'alkylèneglycols en $C_{1-4}$, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.

Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.

les époxyacrylates tels que ceux commercialisés par exemple sous les dénominations SR 349, SR 601, CD 541, SR 602, SR 9036, SR 348, CD 540, SR 480, CD 9038 par le société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER® 3005 et PHOTOMER® 3082 par la société HENKEL.

les poly(méth)acrylates d'(alkyle en $C_{1-50}$) comportant au moins deux fonctions à double liaison éthylénique portées par les chaînes hydrocarbonées latérales et/ou terminales.
De tels copolymères sont commercialisés par exemple sous les dénominations IRR® 375, OTA® 480 et EBECRYL® 2047 par la société UCB.

les polyorganosiloxanes à groupes (méth)acrylate ou (méth)acrylamide.
Des polydiméthylsiloxanes $\alpha,\omega$-diacrylate sont disponibles à la société SHIN-ETSU sous les références X-22-164 B et X-22-164C.

les dendrimères et polymères hyperramifiés portant des groupes terminaux (méth)acrylate ou (méth)acrylamide notamment obtenus respectivement par estérification ou amidification de dendrimères et de polymères hyperramifiés à fonctions terminales hydroxyle ou amino, par de l'acide (méth)acrylique.

Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.

Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).

La société PERSTORP commercialise sous la dénomination BOLTORN® des polyesters hyperramifiés. On trouvera sous la dénomination COMBURST® de la société DENDRITECH des polyéthylèneamines hyperramifiées. Des poly (esteramide) hyperramifiés à extrémités hydroxyle sont commercialisés par la société DSM sous la dénomination HYBRANE®.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes.

Cette fonctionnalité élevée, le plus souvent supérieure à 5, les rend particulièrement utiles en leur permettant de jouer un rôle de "noeud de réticulation", c'est-à-dire de site de réticulation multiple.

Dans un mode de réalisation préféré de l'invention, on utilisera par conséquent ces polymères dendritiques et hyperramifiés en association avec un ou plusieurs des polymères et/ou oligomères a) à h) ci-dessus.

Composés portant au moins deux fonctions à hydrogène(s) labile(s)

Les composés portant au moins deux fonctions à hydrogène labile utilisables dans la présente invention sont également connus. Il peut s'agir de composés organiques de faible masse moléculaire ou bien d'oligomères ou de polymères synthétiques, obtenus par polyaddition, polycondensation et/ou greffage, ou de polymères naturels modifiés chimiquement.

Selon la présente invention, les fonctions à hydrogène labile sont choisies de préférence parmi les fonctions amine primaire (-NH$_2$), amine secondaire (>NH), hydroxyle (-OH), acide carboxylique (-COOH) ou thiol (-SH).

Lorsque la fonction à hydrogène labile est une fonction hydroxyle, on peut citer comme familles de composés, les diols aliphatiques et polyols.

Lorsque la fonction à hydrogène labile est une fonction amine (NH$_2$), il peut s'agir d'une diamine, une polyamine, un aminoalcool, un oligomère, ou un polymère à groupes amines.

Des exemples particuliers de composés porteur de fonctions à hydrogènes labiles sont : les alkylèneglycols en C$_{1-4}$, le glycérol, le triméthylolpropane, le pentaérythritol, les poly(alkylène en C$_{1-4}$)glycols tels que le polyéthylèneglycol ou polyproylèneglycol ou des copolymères de ceux-ci, le produit de condensation de propylèneglycol et de triméthylolpropane, l'huile de ricin, le phytantriol, les sucres et carbohydrates tels que le saccharose ou la cellulose, l'éthylènediamine, le 1,3-diaminopropane, la lysine, l'amino-2-méthyl-2-propanol-1, les poly(alkylèneoxy)diamines telles que les produits JEFFAMINE® commercialisés par la société TEXACO, la nitrocellulose, les esters de cellulose, notamment ceux ayant un degré de substitution inférieur à 3, tels que l'acétobutyrate de cellulose et l'acétopropionate de cellulose, les éthers de cellulose tels que l'hydroxyéthylcellulose, la carboxyméthylcellulose, l'hydroxypropylcellulose ou l'éthylcellulose, les résines polyesters, silicones, perfluoropolyéthers, alkydes et polycétones à extrémités hydroxylées, le poly (alcool vinylique) et les copolymères à base d'alcool vinylique, les copolymères d'alcool allylique, les copolymères à base de (méth)acrylate d'hydroxyalkyle en C$_{2-10}$, comme le (méth)acrylate de 2-hydroxyéthyle ou de 2-hydroxypropyle, vendus notamment sous la dénomination JONCRYL® SCX 910 par la société JOHNSON POLYMER ou sous la dénomination CRODOPLAST® AC 5725 par la société CRODA, les copolymères à base de vinylamine ou d'allylamine, les silicones et les perfluoroéthers à extrémités amine primaire ou secondaire, les dendrimères ou polymères hyperramifiés à extrémités hydroxyle ou amine primaire tels que les polyesters hyperramifiés à extrémités hydroxyle commercialisés par la société PERSTORP sous les dénominations BOLTORN® H40 TMP CORE et HBP POLYOL® 3G (décrits dans les demandes internationales WO 93/17060 et WO 96/12754), ou encore les dendrimères de type polyamido-amines à extrémités amine primaire décrits dans l'article de Tomalia, Angewandte Chemie, Int. Engl. Ed. , vol. 29, n° 2, pages 138 -175.

Selon une seconde variante de l'invention la réticulation est réalisée par voie photochimique et met en oeuvre au moins deux types de composés notamment (A) et (B) possédant respectivement au moins une double liaison insaturée en présence d'un photoamorceur.

Selon cette variante, A et B sont choisi de manière à former un système réactif dont la valence moyenne est supérieure à 2. On appelle valence d'un composé le nombre de liaisons covalentes qu'il peut établir avec les composés qui lui sont associés. On défini la valence moyenne comme étant égale au rapport de la somme des valences de tous les composés A et B divisé par le nombre total de composé A et B

$$V_m = \frac{\sum n_i v_i}{\sum n_i}$$

Selon cette variante de l'invention, les composés A ou B peuvent être un composé comportant une fonction de type doubles liaisons insaturées et notamment tels que définis précédemment, et/ou un monomère à insaturation éthylénique. Un groupe particulier de photoamorceurs avantageux selon l'invention est celui des photoamorceurs copolymérisables. Il s'agit de molécules comportant à la fois un groupe photoamorceur capable d'une scission radicalaire photoinduite et au moins une double liaison éthylénique.

Pour obtenir des propriétés de tenue satisfaisantes, on utilisera généralement une quantité totale de photoamorceur(s) au moins égale à 0,1 % en poids et au plus égale à 10 % en poids, et de préférence comprise entre 0,2 % et 5 % en poids, rapportée au poids total de composés comportant des doubles liaisons éthyléniques.

Dans cette variante, la réticulation peut être effectuée en présence d'un co-filmogène comme par exemple de la nitrocellulose ou des esters de cellulose.

**b) Film polymérique issu de l'évaporation de la phase solvant, organique ou aqueuse, d'une solution ou dispersion d'au moins un polymère filmogène.**

Selon une seconde variante de l'invention, le film polymérique est obtenu par évaporation de la phase solvant, organique ou aqueuse, d'une solution ou dispersion d'au moins un polymère filmogène.

Pour préparer un film polymérique selon l'invention, on peut utiliser un seul polymère filmogène ou un mélange de polymères filmogènes. Ce polymère filmogène peut être choisi dans le groupe constitué par les polymères radicalaires, les polycondensats et les polymères d'origine naturelle.

Ce film peut être obtenu par application de la seconde composition sur un support recouvert de Téflon® puis séchage à une température allant de 20 à 150°C. Le film est ensuite détaché du support.

Polymères filmogènes solubles ou dispersibles dans un solvant organique

Selon une première variante de l'invention, ledit film polymérique dérive de l'évaporation de la phase solvant organique d'une solution ou dispersion d'au moins un polymère filmogène. Dans ce mode de réalisation, le polymère filmogène organique est au moins un polymère choisi parmi le groupe comprenant : les polymères filmogènes solubles ou dispersibles dans au moins une classe de solvant organique tel que par exemple les cétones, les alcools, les glycols et éthers de propylène glycols, les esters à chaînes courtes, les alcanes et leurs mélanges aqueux ou non.

Les polymères correspondants peuvent être de toute nature chimique. En particulier, ils peuvent résulter soit de l'homo- ou co-polymérisation de monomères insaturés, soit de polycondensation, soit de la modification de polymères naturels, en particulier des polysaccharides. Les masses moléculaires moyennes en poids (Mp) de ces polymères peuvent varier de 3 000 à 1 000 000, notamment de 5 000 à 800 000, et en particulier de 10 000 à 500 000.

Parmi les polymères solubles ou dispersibles dans les solvants organiques, les polymères suivants conviennent tout particulièrement :

a) les homo- et co-polymères esters et/ou amides d'acides (méth)acryliques, en particulier les polymères résultant de la polymérisation ou copolymérisation des acrylates et/ou méthacrylates de méthyle, éthyle, propyle, butyle, isobutyle, tertiobutyle, pentyle, hexyle, cyclohexyle, éthyl 2-hexyle, heptyle, octyle, isobornyle, norbornyle, adamantyle, ou les (méth)acrylamides correspondants. Ces polymères comporteront de préférence de 0 à 20 % d'un comonomère polaire tel que acide (méth)acrylique, (méth)acrylamide, (méth)acrylate d'hydroxyéthyle, (méth)acrylate de 2-hydroxypropyle, et le (méth)acrylonitrile. Ils peuvent également résulter de la copolymérisation avec le styrène ou un styrène substitué.

b) Les homo- et co-polymères esters ou amides vinyliques, en particulier les homo- et co-polymères résultant de la polymérisation de l'acétate de vinyle, propionate de vinyle, versatate de vinyle, avec ou sans présence d'un comonomère polaire tel que les acides crotonique, acide allyloxyacétique, anhydride (ou acide) maléique, anhydride (ou acide) itaconique, vinyl acétamide et vinyl formamide. De même, ils peuvent résulter de la copolymérisation d'au moins un des monomères cités avec du styrène ou un styrène substitué.

c) Les celluloses et dérivés cellulosiques comme les nitrocelluloses et/ou les esters de cellulose tels que les acétates de cellulose, les propionates de cellulose, les butyrates de cellulose, les acétopropionates de cellulose et les acétobutyrates de cellulose.

d) Les polycondensats solubles ou dispersibles dans ces solvants. Ils sont généralement utilisés comme filmogène principal ou bien comme cofilmogène d'une des classes de polymères cités précédemment (a à c), en particulier s'ils sont de faible poids moléculaire (Mp < 20 000). Ils peuvent être choisis parmi les polymères ou copolymères suivants : les polyuréthanes, les polyuréthanes acryliques, les polyurées, les polyuréthanes de polyurée, les polyuréthanes de polyester, les polyuréthanes de polyéther, les polyesters, les polyester-amides, les polyesters à chaîne grasse, les époxys, et les condensats arylsulfonamide et en particulier tosylamide/formaldéhydes,

Parmi ces polycondensats, en particulier si on les utilise comme filmogène ou cofilmogène d'une ou plusieurs nitrocelluloses et/ou d'un ester de cellulose (classe c), on peut plus particulièrement citer :

les polyesters, en particulier les polyesters à chaîne grasse et plus particulièrement les copolymères de nom CTFA : « copolymère d'anhydride phtalique/glycérol/décanoate de glycidyle » et « copolymère d'acide adipique/néopentylglycol/anhydride trimellitique »
les alkydes,
les condensats tosylamide/formaldéhyde,
les polyuréthanes et polyurée-uréthans
les résines acryliques.
les résines de silicone (non volatiles ou partiellement volatiles)

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Avantageusement, les première et deuxième séquences et du polymère séquencé sont incompatibles l'une avec l'autre. De tels polymères sont décrits par exemple dans les documents EP1411069 ou WO04/028488.

Dispersions aqueuses de particules de polymères ou encore latex filmogènes

Selon une seconde variante de l'invention, ledit film polymérique dérive de l'évaporation de la phase aqueuse d'une dispersion aqueuse de particules de polymère(s) filmogène(s). Dans ce cas, le polymère filmogène peut être choisi parmi les dispersions aqueuses de particules de polymères ou encore latex filmogènes, et dans ce cas la composition selon l'invention comprend au moins une phase aqueuse.
La dispersion aqueuse comprenant un ou plusieurs polymères filmogènes peut être préparée par l'homme du métier sur la base de ses connaissances générales, notamment par polymérisation en émulsion ou par mise en dispersion du polymère préalablement formé.
Parmi les polymères filmogènes de ce type utilisables dans la composition selon la présente invention, on peut citer les polymères synthétiques, de type polycondensat ou de type radicalaire, les polymères d'origine naturelle, et leurs mélanges.
On peut notamment utiliser, mais sous forme de latex, les polymères (homo- et co-polymères) qui sont cités précédemment comme polymères solubles ou dispersibles en milieu solvant organique, et plus particulièrement les polymères des classes a, b et c.
On peut ainsi citer, parmi les polycondensats, les polyuréthanes anioniques, cationiques, non ioniques ou amphotères, les polyuréthanes-acryliques, les polyuréthanes-polyvinylpyrrolidones, les polyester-polyuréthanes, les polyéther-polyuréthanes, les polyurées, les polyurée polyuréthanes, et leurs mélanges.
On peut également citer les polyesters, les polyesters amides, les polyesters à chaîne grasse, les polyamides et les résines époxyesters.
Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylot propane.
Les polymères de type radicalaires peuvent être notamment des polymères, ou des copolymères, acryliques et/ou vinyliques. On utilise de préférence des polymères radicalaires anioniques. Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.

Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.

Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.

On peut également utiliser des copolymères acryliques/silicones.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

La dispersion peut également comprendre un polymère associatif de type polyuréthane ou une gomme naturelle, telle que la gomme xanthane.

Comme polymère en dispersion aqueuse, on peut citer les dispersions de polymères acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société ZENECA, DOW LATEX 432®, par la société DOW CHEMICAL. On peut également employer des dispersions aqueuses de polyuréthane, et notamment les polyester-polyuréthanes vendues sous les dénominations « AVALURE UR-405® », « AVALURE UR-41041® », « AVALURE UR-425® », « SANCURE 2060® » par la société GOODRICH et les polyéther-polyuréthanes vendus sous les dénominations « SANCURE 878® » par la société GOODRICH, « NEOREZ R-970® » par la société AVECIA.

L'ensemble des polymères filmogènes précités peut être associé à au moins un agent auxiliaire de filmification. L'agent auxiliaire de filmification peut être choisi parmi tous les composés connus de l'homme du métier comme étant susceptibles de remplir la fonction recherchée, et être notamment choisi parmi les agents plastifiants et les agents de coalescence du polymère filmogène.

En particulier, on peut citer, seuls ou en mélange, les plastifiants ou agents de coalescence usuels, tels que:

les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
les esters de glycol,
les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
les esters d'acides notamment carboxyliques tels que les citrates, notamment le citrate de triéthyle, le citrate de tributyle, l'acétylcitrate de triéthyle, l'acétylcitrate de tributyle, l'acétylcitrate de triéthyl-2 hexyle ; les phtalates, notamment le phtalate de diéthyle, le phtalate de dibutyle, le phtalate de dioctyle, le phtalate de dipentyle, le phtalate de diméthoxyéthyle ; les phosphates, notamment le phosphate de tricrésyle, le phosphate de tributyle, le phosphate de triphényle, le phosphate de tributoxyéthyle ; les tartrates, notamment le tartrate de dibutyle ; les adipates ; les carbonates ; les sébaçates ; le benzoate de benzyle, l'acétylricinoléate de butyle, l'acétylricinoléate de glycéryle, le glycolate de butyle, le camphre, le triacétate de glycérol, le N-éthyl-o,p-toluènesulfonamide,
les dérivés oxyéthylénés tels que les huiles oxyéthylénées, notamment les huiles végétales telles que l'huile de ricin; les huiles de silicone,
leurs mélanges.

Le type et la quantité d'agent plastifiant et/ou de coalescence peuvent être choisis par l'homme du métier sur la base de ses connaissances générales.

Par exemple, la teneur en agent plastifiant et/ou de coalescence peut aller de 0,01 % à 20% et en particulier de 0,5 % à 10 % en poids par rapport au poids total de la composition.

Selon un mode de réalisation particulier, le kit de maquillage selon l'invention comprend :

i) une première composition liquide comprenant une phase solvant organique et au moins un polymère filmogène,
ii) un film polymérique souple qui dérive de l'évaporation de la phase solvant, organique ou aqueuse, d'une solution ou dispersion d'au moins un polymère filmogène, ledit film et ladite première composition liquide étant tels que lorsque le film est appliqué sur l'ongle préalablement revêtu de ladite première composition, le film adhère à l'ongle.

Les polymères filmogènes de la première composition et du film polymérique peuvent être identiques ou différents.

Selon un mode de réalisation particulier, le film polymérique souple est un film multicouche réalisé en plusieurs étapes à partir de différentes compositions dérivant de la réticulation d'une composition réticulable et/ou de l'évaporation de la phase solvant, organique ou aqueuse, d'une solution ou dispersion d'au moins un polymère filmogène.

Plus précisément, il peut s'agir d'un film multicouche réalisé par superposition d'au moins deux voire plus de couches obtenues respectivement par évaporation de la phase solvant, organique ou aqueuse, de solutions ou dispersions de polymère(s) filmogène(s) de natures différentes.

## III/ AUTRES ADDITIFS

### i) Pigments et colorants

La première composition et/ou le film polymérique souple peuvent en outre comprendre notamment au moins une matière colorante, organique ou inorganique, notamment de type pigments ou nacres classiquement utilisée dans les compositions cosmétiques.

Par pigments, il faut comprendre des particules blanches ou colorées, minérales ou organiques, insolubles dans le milieu ou aqueux, destinées à colorer et/ou opacifier le film résultant.

Les pigments peuvent être présents à raison de 0,01 à 20 % en poids, notamment de 0,01 à 15 % en poids, et en particulier de 0,02 à 10 % en poids, par rapport au poids total de la première composition et/ou du film polymérique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/ dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La première composition et/ou le film polymérique selon l'invention peuvent comprendre également des colorants hydrosolubles ou liposolubles en une teneur allant de 0,01 à 10 % en poids, notamment allant de 0,01 à 5 % en poids par rapport au poids total de la première composition ou du film polymérique. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Les colorants hydrosolubles sont par exemple le jus de betterave, le bleu de méthylène.

### ii) Matériau à effet

La première composition et/ou le film polymérique selon l'invention peuvent contenir au moins un matériau à effet optique spécifique, notamment présent dans le film polymérique souple. Cet effet est différent d'un simple effet de teinte conventionnel, c'est-à-dire unifié et stabilisé tel que produit par les matières colorantes classiques décrite plus haut comme par exemple les pigments monochromatiques. Au sens de l'invention, « stabilisé » signifie dénué d'effet de variabilité de la couleur avec l'angle d'observation ou encore en réponse à un changement de température.

Ce matériau est présent en quantité suffisante pour produire un effet optique perceptible à l'oeil nu. Avantageusement, il s'agit d'un effet choisi parmi les effets goniochromatique, métallique et notamment miroir, soft-focus, arc en ciel et/ou thermochrome.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

### Particules à reflet métallique

Par « particules à reflet métallique », on désigne des particules dont la nature, la taille, la structure et l'état de surface leur permettent de réfléchir la lumière incidente notamment de façon non iridescente.

Les particules présentant une surface extérieure sensiblement plane conviennent également, car elles peuvent plus facilement donner naissance, si leur taille, leur structure et leur état de surface le permettent, à une réflexion spéculaire intense que l'on peut alors qualifier d'effet miroir.

Les particules à reflet métallique utilisables dans l'invention, peuvent par exemple réfléchir la lumière dans toutes les composantes du visible sans absorber de manière significative une ou plusieurs longueurs d'ondes. La réflectance spectrale de ces particules peut par exemple être supérieure à 70 % dans l'intervalle 400-700 nm, et mieux d'au moins 80 %, voire 90 % ou encore 95 %.

Ces particules ont généralement une épaisseur inférieure ou égale à 1 $\mu$m, notamment inférieure ou égale à 0,7 $\mu$m, en particulier inférieure ou égale à 0,5 $\mu$m.

La proportion totale en particules à reflet métallique est notamment inférieure ou égale à 20 % en poids et en particulier inférieure ou égale à 10 % en poids par rapport au poids total de la première composition ou du film polymérique.

Les particules à reflet métallique utilisables dans l'invention sont en particulier choisies parmi:

les particules d'au moins un métal et/ou d'au moins un dérivé métallique,
les particules comportant un substrat, organique ou minéral, monomatière ou multimatériaux, recouvert au moins partiellement par au moins une couche à reflet métallique comprenant au moins un métal et/ou au moins un dérivé métallique, et
les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

Parmi les dérivés métalliques pouvant être présents dans lesdites particules, on peut citer notamment les oxydes métalliques tels que par exemple les oxydes de titane, notamment $TiO_2$, de fer notamment $Fe_2O_3$, d'étain, de chrome, le sulfate de baryum et les composés suivants : $MgF_2$, $CrF_3$, $ZnS$, $ZnSe$, $SiO_2$, $Al_2O_3$, $MgO$, $Y_2O_3$, $SeO_3$, $SiO$, $HfO_2$, $ZrO_2$, $CeO_2$, $Nb_2O_5$, $Ta_2O_5$, $MoS_2$ et leurs mélanges ou alliages.

Selon une première variante, les particules à reflet métallique peuvent être composées d'au moins un métal tel que défini précédemment, d'au moins un dérivé métallique tel que défini précédemment ou bien encore d'un de leurs mélanges.

Ces particules peuvent être au moins partiellement recouvertes par une couche d'un autre matériau, par un exemple de matériau transparent tel que notamment du colophane, de la silice, des stéarates, des polysiloxanes, des résines polyesters, des résines époxydiques, des résines polyuréthanes et des résines acryliques.

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

 Selon une seconde variante, ces particules peuvent être des particules comportant un substrat et qui présentent donc une structure multicouche par exemple bicouche. Ce substrat peut être organique ou minéral, naturel ou synthétique, monomatière ou multimatériaux, plein ou creux. Lorsque le substrat est synthétique, il peut être réalisé avec une forme favorisant la formation d'une surface réfléchissante après revêtement, notamment après le dépôt d'une couche de matériaux à reflet métallique. Le substrat peut, par exemple, présenter une surface plane et la couche de matériaux à reflet métallique une épaisseur sensiblement uniforme.

Le substrat peut être en particulier choisi parmi les métaux et les dérivés métalliques tels que cités précédemment, et également parmi les verres, les céramiques, les alumines, les silices, les silicates et notamment aluminosilicates et les borosilicates, le mica synthétique tel que le fluorophlogopite, et leurs mélanges, cette liste n'étant pas limitative.

La couche à reflet métallique peut enrober en totalité ou en partie le substrat et cette couche peut être au moins partiellement recouverte par une couche d'un autre matériau, par exemple un matériau transparent notamment tel que cité précédemment. Selon un mode de réalisation particulier, cette couche à reflet métallique enrobe en totalité, directement ou indirectement, c'est-à-dire avec interposition d'au moins une couche intermédiaire métallique ou non, le substrat.

Les métaux ou dérivés métalliques pouvant être utilisés dans la couche réfléchissante sont tels que définis précédemment. Par exemple, elle peut être formée d'au moins un métal choisi parmi l'argent, l'aluminium, le chrome, le nickel, le molybdène, l'or, le cuivre, l'étain, le magnésium et leurs mélanges (alliages). On utilise plus particulièrement l'argent, le chrome, le nickel, le molybdène, et leurs mélanges.

A titre illustratif de ce second type de particules on peut plus particulièrement citer :

Des particules de verre recouvertes d'une couche métallique notamment celles décrites dans les documents JP-A-09188830, JP-A-10158450, JP-A-10158541, JP-A-07258460 et JP-A-05017710.

A titre illustratif de ces particules comportant un substrat de verre, on peut citer celles revêtues respectivement d'argent, d'or ou de titane, en forme de plaquettes, commercialisées par la société NIPPON SHEET GLASS sous les dénominations MICROGLASS METASHINE. Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société. Celles revêtues soit d'oxyde de fer brun, soit d'oxyde de titane, d'oxyde d'étain ou d'un de leur mélange comme celles commercialisées sous la dénomination REFLECKS® par la société ENGELHARD ou celles commercialisées sous la référence METASHINE MC 2080GP par la société NIPPON SHEET GLASS.

Ces particules de verre recouvertes de métaux peuvent être enrobés de silice comme celles commercialisées sous la dénomination METASHINE série PSS1 ou GPS1 par la société NIPPON SHEET GLASS.

Des particules à substrat de verre sphérique revêtu ou non par un métal, notamment celles vendues sous la dénomination PRIZMALITE MICROSPHERE par la société PRIZMALITE INDUSTRIES.

Conviennent également à l'invention, les pigments de la gamme METASHINE 1080R commercialisée par la société NIPPON SHEET GLASS CO. LTD. Ces pigments, plus particulièrement décrits dans la demande de brevet JP 2001-11340, sont des paillettes de verre C-GLASS comprenant 65 à 72 % de SiO2, recouvertes d'une couche d'oxyde de titane de type rutile (TiO2). Ces paillettes de verre ont une épaisseur moyenne de 1 micron et une taille moyenne de 80 microns soit un rapport en taille moyenne/épaisseur moyenne de 80. Elles présentent des reflets bleus, verts, jaunes ou de teinte argent selon l'épaisseur de la couche de TiO2.

Des particules comportant un substrat de borosilicate enrobé d'argent, encore appelées « nacres blanches ».

Des particules à substrat métallique tel que l'aluminium, le cuivre, le bronze, en forme de plaquettes, sont vendues sous la dénomination commerciale STARBRITE par la société SILBERLINE et sous la dénomination VISIONAIRE par la société ECKART.

Des particules comportant un substrat de mica synthétique revêtu de dioxyde de titane, et par exemple les particules de dimension comprise entre 80 et 100 $\mu$m, comportant un substrat de mica synthétique (fluorophlogopite) revêtu de dioxyde de titane représentant 12% du poids total de la particule, vendues sous la dénomination PROMINENCE par la société NIHON KOKEN.

Les particules à reflet métallique peuvent encore être choisies parmi les particules formées d'un empilement d'au moins deux couches à indices de réfraction différents. Ces couches peuvent être de nature polymérique ou métallique et notamment inclure au moins une couche polymérique.

Ainsi, les particules à effet métallique peuvent être des particules dérivant d'un film polymérique multicouche.

Le choix des matériaux destinés à constituer les différentes couches de la structure multicouche est bien entendu effectué de manière à conférer l'effet métallique souhaité aux particules ainsi formées.

De telles particules sont notamment décrites dans WO 99/36477, US 6 299 979 et US 6 387 498 et plus particulièrement identifiées ci-après dans le chapitre goniochromatique.

Pigments diffractants

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article « Pigments Exhibiting Diffractive Effects » d'Alberto Argoitia and Matt Witzman, 2002, Society of Vacuum coaters, 45th Annual Technical Conference Proceedings 2002.

Le pigment diffractant peut être réalisé avec des motifs ayant différents profils, notamment triangulaires, symétriques ou non, en créneaux, de largeur constante ou non, sinuosoïdaux.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non.

Une structure possible pour le pigment diffractant peut comporter une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : $MgF_2$, $SiO_2$, $Al_2O_3$, $AlF_3$, $CeF_3$, $LaF_3$, $NdF_3$, $SmF_2$, $BaF_2$, $CaF_2$, $LiF$ et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs composés, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : $MgF_2$, $SiO$, $SiO_2$, $Al_2O_3$, $TiO_2$, $WO$, $AlN$, $BN$, $B_4C$, $WC$, $TiC$, $TiN$, $N_4Si_3$, $ZnS$, des particules de verre, des carbones de type diamant et leurs associations.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante : $MgF_2/Al/MgF_2$, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du $MgF_2$ peut être comprise entre 80 et 95 % du poids total du pigment.

Agents de coloration goniochromatiques

Au sens de l'invention, un agent de coloration goniochromatique permet d'observer un changement de couleur, encore appelé « color flop », en fonction de l'angle d'observation, supérieur à celui que l'on peut rencontrer avec des nacres. On peut utiliser simultanément un ou plusieurs agents de coloration goniochromatique.

L'agent de coloration goniochromatique peut être choisi de manière à présenter un changement de couleur relativement important avec l'angle d'observation.

L'agent de coloration goniochromatique peut ainsi être choisi de telle sorte que l'on puisse observer, pour une variation de l'angle d'observation comprise entre 0° et 80° sous un éclairage à 45°, une variation de couleur $\Delta E$ de la composition cosmétique, mesurée dans l'espace colorimétrique CIE 1976, d'au moins 2.

L'agent de coloration goniochromatique peut également être choisi de telle sorte que l'on puisse observer, pour un éclairage à 45° et une variation de l'angle d'observation comprise entre 0° et 80°, une variation Dh de l'angle de teinte de la composition cosmétique, dans le plan CIE 1976, d'au moins 30° voire au moins 40° ou au moins 60°, voire encore d'au moins 100°.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

La structure multicouche peut présenter ou non, par rapport à une couche centrale, une symétrie au niveau de la nature chimique des couches empilées.

Des exemples de structures multicouche interférentielles symétriques utilisables dans des compositions réalisées conformément à l'invention sont par exemple les structures suivantes : $Al/SiO_2/Al/SiO_2/Al$, des pigments ayant cette structure étant commercialisés par la société DUPONT DE NEMOURS ; $Cr/MgF_2/Al/MgF_2/Cr$, des pigments ayant cette structure étant commercialisés sous la dénomination CHROMAFLAIR par la société FLEX ; $MoS_2/SiO_2/Al/SiO_2/MoS_2$ ; $Fe_2O_3/SiO_2/Al/SiO_2/Fe_2O_3$, et $Fe_2O_3/SiO_2/Fe_2O_3/SiO_2Fe_2O_3$, des pigments ayant ces structures étant commercialisés sous la dénomination SICOPEARL par la société BASF ; $MoS_2/SiO_2/mica-oxyde/SiO_2/MoS_2$ ; $Fe_2O_3/SiO_2/mica-oxyde/SiO_2/Fe_2O_3$ ; $TiO_2/SiO_2/TiO_2$ et $TiO_2/Al_2O_3/TiO_2$ ; $SnO/TiO_2/SiO_2/TiO_2/SnO$ ; $Fe_2O_3/SiO_2/Fe_2O_3$ ; $SnO/mica/TiO_2/SiO_2/TiO_2/mica/SnO$, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure $Fe_2O_3/SiO_2/Al/SiO_2/Fe_2O_3$ on passe du doré-vert au gris-rouge pour des couches de $SiO_2$ de 320 à 350 nm ; du rouge au doré pour des couches de $SiO_2$ de 380 à 400 nm ; du violet au vert pour des couches de $SiO_2$ de 410 à 420 nm ; du cuivre au rouge pour des couches de $SiO_2$ de 430 à 440 nm.

On peut encore utiliser des agents de coloration goniochromatiques à structure multicouche comprenant une alternance de couches polymériques.

A titre illustratif des matériaux pouvant constituer les différentes couches de la structure multicouche, on peut citer, cette liste n'étant pas limitative: le naphthalate de polyéthylène (PEN) et ses isomères par exemple 2,6-, 1,4-, 1,5-, 2,7- et 2,3-PEN, les téréphthalates de polyalkylene, des polyimides, des polyéthérimides, des polystyrènes atactiques, des polycarbonates, des polyméthacrylates et des polyacrylates d'alkyle, du polystyrène syndiotactique (sPS), des poly-alpha-méthylstyrène syndiotactiques, du polydichlorostyrène syndiotactique, copolymères et mélange de ses polystyrènes, des dérivés de cellulose, des polymères polyalkylènes, des polymères fluorés, des polymères chlorés, des polysulfones, des polyéthersulfones, des polyacrylonitriles, des polyamides, des résines siliconées, des résines époxy, de l'acétate de polyvinyle, des polyéthers-amides, des résines ionomériques, des élastomères et polyuréthanes. Conviennent également des copolymères, par exemple copolymères de PEN (par exemple, copolymères de 2,6-, 1,4-, 1,5-, 2,7-, et/ou 2,3-acide naphtalène dicarboxylique ou ses esters avec (a) acide téréphtalique ou ses esters ; (b) l'acide isophtalique ou ses esters ; (c) acide phtalique ou ses esters ; (d) des alcanes glycols ; (e) des cycloalkanes glycols (par exemple le cyclohexane diméthanol diol) ; (f) des acides alcanes dicarboxyliques ; et/ou (g) des acides cycloalkanes dicarboxylique, des copolymères de téréphthalates de polyalkylène et des copolymères de styrène. En outre, chaque couche individuelle peut inclure des mélanges de deux ou plusieurs polymères ou copolymères précédents. Le choix des matériaux destinés à constituer les différentes couches de la structure multicouche est bien entendu effectué de manière à conférer l'effet optique souhaité aux particules ainsi formées.

On peut citer, à titre d'exemple de pigments à structure multicouche polymérique, ceux commercialisés par la société 3M sous la dénomination COLOR GLITTER.

Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société CHENIX ainsi que celle commercialisées sous la dénomination HELICONE® HC par la société WACKER.

Ces agents peuvent également être sous forme de fibres goniochromatiques dispersées. De telles fibres peuvent par exemple présenter une taille comprise entre 50 $\mu$m et 700 $\mu$m, par exemple d'environ 300 $\mu$m.

En particulier, on peut utiliser des fibres interférentielles à structure multicouche. Des fibres à structure multicouche de polymères sont notamment décrites dans les documents EP-A-921217, EP-A-686858 et US-A-5472798. La structure multicouche peut comporter au moins deux couches, chaque couche, indépendamment ou non de la (ou les) autre(s) couche(s), étant réalisée en au moins un polymère de synthèse. Les polymères présents dans les fibres peuvent avoir un indice de réfraction allant de 1,30 à 1,82 et mieux allant de 1,35 à 1,75. Les polymères préférés pour constituer les fibres sont les polyesters tels que le polyéthylène téréphtalate, le polyéthylène naphtalate, le polycarbonate ; les polymères acryliques comme le polyméthacrylate de méthyle ; les polyamides.

Des fibres goniochromatiques à structure bicouche polyéthylène téréphtalate/nylon-6 sont commercialisées par la société TEIJIN sous la dénomination MORPHOTEX.

Dans une variante, cet agent de coloration goniochromatique peut être associé à au moins un pigment diffractant.

La combinaison de ces deux matériaux résulte en uns composition ou un film qui présente une variabilité de la couleur accrue, donc qui est susceptible de permettre à un observateur de percevoir un changement de couleur, voire un mouvement de couleur, dans de nombreuses conditions d'observation et d'illumination.

Le rapport pondéral du pigment diffractant par rapport à l'agent de coloration goniochromatique est de préférence compris entre 85/15 et 15/85, mieux entre 80/20 et 20/80, mieux encore 60/40 et 40/60, par exemple de l'ordre de 50/50. Un tel rapport est favorable à l'obtention d'un effet arc-en-ciel et d'un effet goniochromatique soutenus.

Azurants optiques

Les azurants optiques sont des composés bien connus de l'homme du métier. De tels composés sont notamment décrits dans « Fluorescent Whitening Agent, Encyclopedia of Chemical Technology, Kirk-Othmer », vol 11, p. 227-241, 4ème édition, 1994, Wiley.

On peut plus particulièrement les définir comme des composés qui absorbent essentiellement dans l'UVA entre 300 et 390 nm et réémettent essentiellement entre 400 et 525 nm.

Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et leurs mélanges.

De tels composés sont facilement disponibles commercialement. On peut citer par exemple :

le dérivé stilbénique de naphto-triazole vendu sous la dénomination commerciale « Tinopal GS », le di-styryl-4,4' biphényle sulfonate di-sodique (nom CTFA : disodium distyrylbiphenyl disulfonate) vendu sous la dénomination commerciale « Tinopal CBS-X », le dérivé cationique d'aminocoumarine vendu sous la dénomination commerciale « Tinopal SWN CONC. », le 4,4'-bis[(4,6-dianilino-1,3,5-triazin-2-yl)amino]stilbène-2,2'-disulfonate de sodium vendu sous la dénomination commerciale « Tinopal SOP », le 4,4'-bis-[(4-anilino-6-bis(2-dydroxyéthyl)amino-1,3,5-triazin-2-yl)amino]stilbène-2,2'-disulfonic acide vendu sous la dénomination commerciale « Tinopal UNPA-GX », le 4,4'-bis-[anilino-6-morpholine-1,3,5-triazin-2-yl)amino]stilbène vendu sous la dénomination commerciale « Tinopal AMS-GX », le 4,4'-bis-[(4-anilino-6-(2-hydroxyéthyl)méthyl amino-1,3,5-triazin-2-yl)amino]stilbène-2,2'-disodium sulfonate vendu sous la dénomination commerciale « Tinopal 5BM-GX », tous par la société CIBA Spécialités Chimiques,

le 2,5 thiophène di-yl bis(5 ter-butyl-1,3 benzoxazole) vendu sous la dénomination commerciale « Uvitex OB » par la société CIBA,

le dérivé anionique du di-aminostilbène en dispersion dans l'eau vendu sous la dénomination commerciale « Leucophor BSB liquide » par la société CLARIANT,

les laques d'azurant optique vendues sous la dénomination commerciale « COVAZUR » par la société WACKHERR.

Les azurants optiques utilisables dans la présente invention peuvent aussi se présenter sous la forme de copolymères, par exemple d'acrylates et/ou de méthacrylates, greffés par des groupements azurants optiques comme décrits dans la demande FR 99 10942.

Ils peuvent être utilisés tels quels ou introduits dans le film sous forme de particules et/ou de fibres recouvertes dudit azurant, telles que celles décrites ci-après.

En particulier, on peut utiliser les fibres recouvertes d'azurant optique telles que commercialisées par la société LCW sous la référence commerciale Fiberlon 54 ZO3, ayant une longueur d'environ 0,4 mm et un titre de 0,5 deniers.

Matériau à effet relief :

L'effet relief peut ou non être associé à un effet optique. Un matériau de ce type est généralement présent en quantité suffisante pour conférer un effet relief perceptible au toucher voire à l'oeil nu. Il peut notamment s'agir d'un effet rugueux et/ou martelé.

Matériau conférant un aspect rugueux

Ainsi, le film polymérique du kit selon l'invention est particulièrement avantageux pour la fixation de particules ou fibres solides au niveau de son film conférant ainsi un maquillage original en relief. De plus, les particules de forme sensiblement sphérique ou ovoïdale peuvent conférer un toucher doux au maquillage.

Avantageusement, les particules solides ont une forme sensiblement sphérique, pour permettre leur bonne répartition lors de leur application sur la première couche.

Les particules solides utilisées selon l'invention peuvent avoir une taille moyenne allant de 2,5 $\mu$m à 5 mm, et mieux de 50 $\mu$m à 2 mm. Plus les particules sont de petites tailles, plus la tenue des particules est satisfaisante. L'emploi de particules est également compatible avec la réalisation de motifs.

Les particules solides peuvent être en tout matériau satisfaisant les propriétés de densités définies précédemment. Par exemple, les particules solides peuvent être en un matériau choisi parmi le verre, l'oxyde de zirconium, le carbure de tungstène, les plastiques comme les polyuréthanes, les polyamides, le polytétrafluoroéthylène, le polypropylène, les métaux comme l'acier, le cuivre, le laiton, le chrome, le marbre, l'onyx, le jade, la nacre naturelle, les pierres précieuses (diamant, émeraude, rubis, saphir), l'améthyste, l'aigue-marine. On utilise de préférence des billes de verres telles que celles vendues sous la dénomination "SILIBEADS®" par la société SIGMUND LINDNER ; ces billes ont en outre pour avantage de conférer également un effet brillant et un scintillement au maquillage.

Les particules solides, déformables ou non, peuvent être pleines ou creuses, incolores ou colorées, enrobées ou non.

En ce qui concerne les fibres utilisables selon l'invention, il peut s'agir de fibres d'origine synthétique ou naturelle, minérale ou organique.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Il peut notamment s'agir de fibres utilisées dans la fabrication des textiles et notamment des fibres de soie, de coton, de laine, de lin, des fibres de cellulose, notamment extraite du bois, des légumes ou des algues, de rayonne, de polyamide (Nylon®), de viscose, d'acétate notamment d'acétate de rayonne, de poly-(p-phénylène-téréphtalamide) (ou d'aramide) notamment de Kevlar®, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

Matériau conférant un aspect martelé

Les inventeurs ont également constaté qu'il était possible de conditionner dans le film polymérique du kit selon l'invention un matériau comprenant un mélange silice pyrogéné, pigment métallique et composé organopolysiloxane pour lui conférer un aspect martelé.

Un tel mélange est notamment décrit dans la demande de brevet EP 1 040 813.

Matériau à effet olfactif

Avantageusement, le film polymérique selon l'invention peut également être doté de propriétés olfactives par incorporation notamment dans ledit film d'au moins un matériau odorant ou encore substance parfumante.

La substance parfumante peut être choisie parmi toute substance odoriférante bien connue de l'homme du métier, et notamment parmi les huiles essentielles et/ou les essences.

Ce matériau olfactif peut, si nécessaire, être introduit via un solvant-plastifiant.

On entend par « solvant-plastifiant » un composé qui solubilise au moins partiellement le matériau olfactif et qui est susceptible de s'évaporer lentement.

Le solvant-plastifiant peut être choisi parmi des glycols tels que le dipropylène glycol, l'éthyldiglycol, le n-propylglycol, le n-butylglycol, le méthyldiglycol, le n-butyldiglycol, des alcools tels que le cyclohexanol, l'éthyl-2 butanol, le méthoxy-3 butanol, l'éthyl-2 hexanol, le phénoxyéthanol, des esters, tels que le monoacétate de glycol, l'acétate d'éthylglycol, l'acétate de n-butylglycol, l'acétate d'éthyldiglycol, l'acétate de n-butyldiglycol, l'abiétate de méthyle, le myristate d'isopropyle, le diacétate de propylène glycol, l'acétate d'éther méthylique du propylène glycol, des éthers de glycols tels que l'éther méthylique du dipropylène glycol, l'éther butylique du dipropylène glycol, seuls ou en mélange.

La première composition et/ou le film polymérique souple peuvent également contenir un ou plusieurs additifs de formulation couramment utilisés en cosmétique et plus spécialement dans le domaine cosmétique et/ou soin des ongles. Ils peuvent notamment être choisis parmi les vitamines, les oligo-éléments, les adoucissants, les séquestrants, les agents alcalinisants ou acidifiants, les agents mouillants, les agents épaississants, les agents dispersants, les anti-mousses, les agents d'étalement, les co-résines, les conservateurs, les filtres UV, les actifs, les agents hydratants, les neutralisants, les stabilisants, les antioxydants et leurs mélanges.

Ainsi, il peut notamment incorporer, à titre d'actifs, des agents durcissants ou renforcateur pour matières kératiniques, des actifs favorisant la pousse de l'ongle tel que le méthylsulfonylméthane et/ou des actifs pour traiter des affections diverses localisées au niveau de l'ongle, comme par exemple l'onichomycose.

Les quantités de ces différents ingrédients sont celles classiquement utilisées dans ce domaine et par exemple de 0,01 à 20 % en poids, et notamment de 0,01 à 10 % en poids par rapport au poids total de la première composition ou du film polymérique.

L'exemple qui suit illustre de manière non limitative l'invention.

## Exemple 1 : Kit de maquillage des ongles

### 1) Première composition liquide

| | | |
|---|---|---|
| Nitrocellulose | | 10% |
| Plastifiant : -n-éthyl-o,p-toluène-sulfonamide (Resimpol 8 de PAN-AMERICANA) | | 4% |
| Resine alkyde | | 10% |
| Acétate de butyle/acétate d'éthyle 50/50 | qsp | 100 |

### 2) Film polymérique souple

| | | |
|---|---|---|
| Nitrocellulose | | 10% |
| Plastifiant : -n-éthyl-o,p-toluène-sulfonamide (Resimpol 8 de PAN-AMERICANA) | | 5% |
| Resine alkyde | | 10% |
| Hectorite modifiée (Bentone 27V d'Elementis) | | 1,3% |
| Pigments | | 1% |
| Acétate de butyle/acétate d'éthyle 50/50 | qsp | 100 |

On étale une couche d'une épaisseur de 600 ± 20 microns de la composition ci-dessus sur une plaque recouverte de Téflon® et on laisse sécher 7 jours à température ambiante (25°C). On obtient un film polymérique souple d'épaisseur environ 130 ± 30 microns issu de l'évaporation de la phase solvant de la composition, que l'on détache du support.

On applique ensuite sur l'ongle une couche de la première composition liquide 1), on attend quelques secondes, puis on applique sur l'ongle revêtu de la première composition le film polymérique souple 2) préalablement découpé à la forme de l'ongle. L'excès de film est découpé à la taille de l'ongle avec un instrument coupant.

**Revendications**

1. Kit de maquillage ou de soin des ongles comprenant :

   i) une première composition liquide
   ii) un film polymérique souple, ledit film et ladite première composition liquide étant tels que lorsque le film est appliqué sur l'ongle préalablement revêtu de ladite première composition, le film adhère à l'ongle.

2. Kit de maquillage selon la revendication 1 **caractérisé en ce que** le film polymérique dérive de la réticulation d'une composition réticulable et/ou de l'évaporation de la phase solvant, organique ou aqueuse, d'une solution ou dispersion d'au moins un polymère filmogène.

3. Kit selon la revendication 1 ou 2, **caractérisé en ce que** la première composition comprend un matériau adhésif et une phase solvant organique.

4. Kit selon la revendication 3, **caractérisé en ce que** ledit matériau adhésif est choisi parmi les copolymères dérivant de la copolymérisation de monomères vinyliques avec des entités polymériques, des copolymères possédant un squelette polymérique, de Tg variant de 0 °C à 45 °C, greffés par des chaînes dérivant de monomères acryliques et/ou méthacryliques et possédant une Tg variant de 50 °C à 200 °C et les polyisobutylènes présentant une masse molaire relative Mv supérieure ou égale à 10 000 et inférieure ou égale à 150 000.

5. Kit selon la revendication 1 ou 2, **caractérisé en ce que** la première composition comprend une phase solvant et un polymère filmogène.

6. Kit selon l'une des revendications précédentes, **caractérisé en ce que** le film polymérique possède un extrait sec supérieur à 80% et notamment supérieur ou égal à 85 % en poids.

7. Kit selon l'une des revendications précédentes, **caractérisé en ce que** le film polymérique dérive de la réticulation par voie thermique, photochimique et/ou chimique d'une composition réticulable.

8. Kit selon la revendication 7, **caractérisé en ce que** ladite composition comprend au moins un système réactif formé par :

   - au moins un premier composé (A) comprenant au moins deux fonctions (X),
   - au moins un deuxième composé (B) comprenant au moins deux fonctions (Y), réactives vis-à-vis des fonctions X, ledit système réactif possédant une fonctionnalité moyenne (nombre total des fonctions X et Y/nombre total des molécules de composés (A) et (B)) supérieure à 2.

9. Kit selon la revendication 7 ou 8, **caractérisé en ce que** ladite réticulation est de type polyaddition et/ou polycondensation de composé(s) comprenant au moins deux fonctions isocyanates et/ou époxydes avec des composés possédant au moins deux fonctions à hydrogène(s) labile(s).

10. Kit selon la revendication 9, **caractérisé en ce que** les composés porteurs de fonctions réactives de type isocyanate sont choisis parmi les diisocyanates, triisocyanates et polyisocyanates aliphatiques, cycloaliphatiques ou aromatiques de masse moléculaire inférieure à 10 000.

11. Kit selon la revendication 7 ou 8, **caractérisé en ce que** ladite réticulation est réalisée par voie photochimique et met en oeuvre au moins deux types de composés portant respectivement au moins une double liaison insaturée, en présence d'un photoamorceur.

12. Kit selon l'une des revendications 1 à 6, **caractérisé en ce que** ledit film polymérique dérive de l'évaporation de la phase solvant d'une solution ou dispersion d'au moins un polymère filmogène.

13. Kit selon la revendication 5 ou 12, **caractérisé en ce que** ledit polymère filmogène est choisi parmi les homo- et co-polymères esters et/ou amides d'acides (méth)acryliques, les homo- et co-polymères esters ou amides vinyliques, les celluloses et dérivés cellulosiques, les polyuréthanes, les polyuréthanes acryliques, les polyurées, les polyuréthanes de polyurée, les polyuréthanes de polyester, les polyuréthanes de polyéther, les polyesters, les polyester-amides, les polyesters à chaîne grasse, les époxys, et les condensats arylsulfonamide.

**14.** Kit selon l'une quelconque des revendications 3, 5, 12 ou 13, **caractérisé en ce que** la phase solvant comprend au moins un solvant organique choisi parmi :

- les esters à chaîne courte (ayant de 3 à 8 atomes de carbone au total), tels que l'acétate d'éthyle, l'acétate de méthyle, l'acétate de propyle, l'acétate de n-butyle, l'acétate d'isopentyle ;
- les cétones liquides à température ambiante, telles que la méthyléthylcétone, la méthylisobutylcétone, la diisobutylcétone, l'isophorone, la cyclohexanone, l'acétone ;
- les alcools liquides à température ambiante, tels que l'éthanol, l'isopropanol, le diacétone alcool, le 2-butoxyéthanol, le cyclohexanol ;
- les glycols liquides à température ambiante, tels que l'éthylène glycol, le propylène glycol, le pentylène glycol, le glycérol ;
- les éthers de propylène glycol liquides à température ambiante, tels que le monométhyléther de propylène glycol, l'acétate de monométhyl éther de propylène glycol, le mono n-butyl éther de dipropylène glycol ;
- les aldéhydes liquides à température ambiante, tels que le benzaldéhyde, l'acétaldéhyde ;
- les carbonates tel que le carbonate de propylène, le carbonate de diméthyle ;
- les acétals tels que le méthylal ; et
- leurs mélanges.

**15.** Kit selon la revendication précédente, **caractérisé en ce que** le ou les solvants organiques représente(nt) de 5 à 95% du poids total de la composition, de préférence de 10% à 85% en poids.

**16.** Kit selon l'une des revendications 1 à 6, **caractérisé en ce que** ledit film polymérique dérive de l'évaporation de la phase aqueuse d'une dispersion aqueuse de particules de polymère(s) filmogène(s).

**17.** Kit selon la revendication 16, **caractérisé en ce que** la dispersion aqueuse de particules de polymère(s) filmogène(s) est un latex, un pseudolatex ou un de leurs mélanges.

**18.** Kit selon la revendication 16 ou 17, **caractérisé en ce que** ledit polymère est choisi parmi les polycondensats, les polyuréthanes anioniques, cationiques, non ioniques ou amphotères, les polyuréthanes-acryliques, les polyuréthanes-polyvinylpyrrolidones, les polyester-polyuréthanes, les polyéther-polyuréthanes, les polyurées, les polyurée polyuréthanes, les polyesters, les polyesters amides, les polyesters à chaîne grasse, les polyamides et les résines époxyesters, les polymères ou copolymères acryliques et/ou vinyliques, les copolymères acryliques/silicones ou nitrocellulose/acryliques, les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters et les polyesteramides et/ou les alkydes.

**19.** Kit selon l'une des revendications précédentes, **caractérisé en ce que** le solvant organique majoritaire de la première composition est susceptible de conduire à une augmentation de masse du film polymérique sec mis à son contact, notamment d'au moins 10%, de préférence 20%, après immersion pendant 60 minutes dudit film dans ledit solvant à température ambiante (25°C).

**20.** Kit selon l'une quelconque des revendications précédentes, **caractérisé en ce que** la première composition et/ou le film polymérique comprend en outre au moins un additif de formulation notamment choisi parmi les co-résines, les plastifiants, les agents de coalescence et les agents d'étalement.

**21.** Kit selon l'une quelconque des revendications précédentes, **caractérisé en ce que** la première composition et/ou le film polymérique souple comprend en outre une quantité efficace d'au moins une matière colorante.

**22.** Kit selon l'une quelconque des revendications précédentes, **caractérisé en ce que** la première composition et/ou le film polymérique souple contient au moins un matériau à effet optique, relief et/ou olfactif.

**23.** Kit selon l'une quelconque des revendications 1 à 20, **caractérisé en ce que** la première composition est transparente.

**24.** Kit selon l'une quelconque des revendications 1 à 20, **caractérisé en ce que** la première composition est exempte de matière colorante et de matériau à effet optique et/ou relief.

**25.** Kit selon l'une quelconque des revendications précédentes, **caractérisé en ce que** film polymérique souple est prédécoupé à la forme et à la taille de l'ongle.

**26.** Kit selon l'une quelconque des revendications 1 à 25, **caractérisé en ce que** le film polymérique est démaquillable par des solvants organiques et notamment par les acétates d'alkyle et leurs mélanges.

**27.** Kit de maquillage ou de soin des ongles comprenant :

i) une première composition liquide comprenant une phase solvant organique et au moins un polymère filmogène,
ii) un film polymérique souple qui dérive de l'évaporation de la phase solvant, organique ou aqueuse, d'une solution ou dispersion d'au moins un polymère filmogène, ledit film et ladite première composition liquide étant tels que lorsque le film est appliqué sur l'ongle préalablement revêtu de ladite première composition, le film adhère à l'ongle.

**28.** Procédé de maquillage ou de soin des ongles **caractérisé en ce qu'**il consiste :

a) à appliquer sur l'ongle au moins une couche d'une première composition liquide,
b) puis, à appliquer sur ladite couche un film polymérique souple apte à adhérer sur l'ongle via ladite première composition liquide.

**29.** Procédé selon la revendication précédente, **caractérisé en ce que** le film polymérique est appliqué sur la couche de première composition liquide après séchage partiel de ladite couche.

**30.** Procédé de maquillage des ongles **caractérisé en ce qu'**il consiste à appliquer sur l'ongle un film polymérique souple dont la face destinée à être en contact avec l'ongle a été préalablement enduite d'au moins une couche d'une première composition liquide, ledit film et ladite première composition liquide étant tels que lorsque le film est appliqué sur l'ongle préalablement revêtu de ladite première composition, le film adhère à l'ongle.

**31.** Procédé selon l'une des revendications 28 ou 30, **caractérisé en ce que** la première composition est conforme à l'une quelconque des revendications 3 à 5.

**32.** Procédé selon l'une des revendications 28 à 31, **caractérisé en ce que** le film polymérique est conforme à l'une quelconque des revendications 2 et 6 à 18.

**33.** Procédé selon l'une des revendications 28 à 32, **caractérisé en ce que** le film polymérique est prédécoupé à la forme de l'ongle.

**34.** Procédé selon l'une des revendications 28 à 33, **caractérisé en ce qu'**il comprend une étape de découpage du film polymérique à la forme et la taille de l'ongle.

**35.** Procédé selon l'une des revendications 28 à 34, **caractérisé en ce qu'**au moins une couche supplémentaire d'une seconde composition liquide comprenant un polymère filmogène et un milieu solvant organique est appliquée sur ledit film polymérique.

**Office européen
des brevets**

**RAPPORT DE RECHERCHE EUROPEENNE**

Numéro de la demande

EP 05 29 1352

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 99/56705 A (THE PROCTER & GAMBLE COMPANY) 11 novembre 1999 (1999-11-11) <br> * page 2, ligne 8 - ligne 23 * <br> * page 3, ligne 24 - ligne 31 * <br> * page 4, ligne 32 - page 5, ligne 24 * <br> * page 7, ligne 20 - ligne 31 * <br> * page 8, ligne 7 - page 10, ligne 4 * <br> * exemples * <br> ----- | 1-35 | A61Q3/02 <br> A61K8/85 |
| X | WO 01/82865 A (REVLON CONSUMER PRODUCTS CORPORATION) 8 novembre 2001 (2001-11-08) <br> * page 2, ligne 5 - ligne 31 * <br> * page 3, ligne 8 - ligne 16 * <br> * page 3, ligne 18 - ligne 34 * <br> * page 9, ligne 26 - page 10, ligne 5 * <br> * page 10, ligne 16 - ligne 35 * <br> * exemples * <br> ----- | 1-35 | |
| X | US 6 197 316 B1 (ELLINGSON PETER CHRISTOPHER ET AL) 6 mars 2001 (2001-03-06) <br> * colonne 1, ligne 54 - ligne 67 * <br> * colonne 2, ligne 45 - ligne 57 * <br> * colonne 3, ligne 24 - colonne 4, ligne 12 * <br> * exemples * <br> ----- | 1-35 | **DOMAINES TECHNIQUES RECHERCHES (IPC)** <br><br> A61K |
| X | US 5 772 988 A (PAGANO ET AL) 30 juin 1998 (1998-06-30) <br> * colonne 3, ligne 18 - colonne 4, ligne 2 * <br> * colonne 4, ligne 7 - ligne 18 * <br> * exemples * <br> ----- | 1-35 | |

Le présent rapport a été établi pour toutes les revendications

| Lieu de la recherche | Date d'achèvement de la recherche | Examinateur |
|---|---|---|
| Munich | 23 novembre 2005 | Ruckebusch, V |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................

& : membre de la même famille, document correspondant

EPO FORM 1503 03.82 (P04C02)

**EP 1 634 621 A1**

## ANNEXE AU RAPPORT DE RECHERCHE EUROPEENNE
## RELATIF A LA DEMANDE DE BREVET EUROPEEN NO.

EP 05 29 1352

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-11-2005

| Document brevet cité au rapport de recherche | | Date de publication | Membre(s) de la famille de brevet(s) | | Date de publication |
|---|---|---|---|---|---|
| WO 9956705 | A | 11-11-1999 | AU | 3876199 A | 23-11-1999 |
| | | | CN | 1299267 A | 13-06-2001 |
| | | | EP | 1083862 A2 | 21-03-2001 |
| | | | JP | 2002513735 T | 14-05-2002 |
| | | | US | 6306375 B1 | 23-10-2001 |
| WO 0182865 | A | 08-11-2001 | AU | 5928001 A | 12-11-2001 |
| | | | CA | 2407205 A1 | 08-11-2001 |
| | | | EP | 1278501 A2 | 29-01-2003 |
| | | | ZA | 200208935 A | 09-12-2003 |
| US 6197316 | B1 | 06-03-2001 | CN | 1299265 A | 13-06-2001 |
| | | | EP | 1073402 A1 | 07-02-2001 |
| | | | JP | 2002513741 T | 14-05-2002 |
| | | | WO | 9956711 A1 | 11-11-1999 |
| US 5772988 | A | 30-06-1998 | AU | 707799 B2 | 22-07-1999 |
| | | | AU | 3062097 A | 05-12-1997 |
| | | | CA | 2226567 A1 | 20-11-1997 |
| | | | EP | 0845973 A1 | 10-06-1998 |
| | | | JP | 11509869 T | 31-08-1999 |
| | | | WO | 9742930 A1 | 20-11-1997 |
| | | | ZA | 9704081 A | 09-12-1997 |

Pour tout renseignement concernant cette annexe : voir Journal Officiel de l'Office européen des brevets, No.12/82